# EUROPÄISCHE PATENTSCHRIFT

(11) **EP 4 021 280 B1**
(45) Veröffentlichungstag und Bekanntmachung des Hinweises auf die Patenterteilung: **17.07.2024**
(21) Anmeldenummer: 20758112.5
(22) Anmeldetag: 13.05.2020
(51) Int. Cl.: A61B 5/00

(54) **VORRICHTUNG ZUR ERZEUGUNG VON ABBILDUNG VON TIEFENABHÄNGIGEN MORPHOLOGISCHEN STRUKTUREN VON HAUTLÄSIONEN**
DEVICE FOR PRODUCING AN IMAGE OF DEPTH-DEPENDENT MORPHOLOGICAL STRUCTURES OF SKIN LESIONS
DISPOSITIF POUR GÉNÉRER UNE REPRÉSENTATION DE STRUCTURES MORPHOLOGIQUES DE LÉSIONS CUTANÉES QUI SONT FONCTION DE LA PROFONDEUR

(30) Priorität: 26.08.2019 DE 102019122843
(43) Veröffentlichungstag der Anmeldung: 06.07.2022
(73) Patentinhaber: Fotofinder Systems GmbH, 84364 Bad Birnbach (DE)
(72) Erfinder: MAYER, Andreas, 84364 Bad Birnbach (DE)
(74) Vertreter: advotec.
(86) Internationale Anmeldenummer: PCT/DE2020/200035
(87) Internationale Veröffentlichungsnummer: WO 2021/037315

(56) Entgegenhaltungen:
- US-A1- 2002 010 394
- US-A1- 2008 186 591
- US-A1- 2010 302 358
- US-A1- 2018 061 046
- US-B2- 9 427 187

## Beschreibung

Die Erfindung betrifft eine Vorrichtung und ein Verfahren zur Erzeugung von Abbildungen zur Unterstützung bei der Charakterisierung von Hautläsionen eines menschlichen Körpers.

Ein aus dem Stand der Technik bekanntes Verfahren zur Erfassung von Hautläsionen, d.h. von Hautveränderungen und Hautschädigungen, ist die Dermatoskopie bzw. die Auflichtmikroskopie, ein nichtinvasives Untersuchungsverfahren bei dem die zu untersuchenden Hautbereiche mit einem Auflichtmikroskop unter Beleuchtung mit polarisiertem Licht mit circa 10 bis 20-facher Vergrößerung betrachtet werden, um die für bestimmte benigne und maligne Läsionstypen typischen Formen und Strukturen zu erkennen und zu beurteilen. Eine Einschätzung bzw. Diagnose erfolgt dann durch den behandelnden Arzt mittels visueller Begutachtung. Diese kann dann mit einer zusätzlichen histologischen Untersuchung des Hautbereichs bestätigt werden, für welche jedoch ein chirurgischer Eingriff zur Entnahme einer Gewebeprobe notwendig ist. Seit Einführung der Computertechnik werden dazu auch digitale Einzelbilder angefertigt und abgespeichert, um sie später zu vergleichen oder auszuwerten.

Ebenso ist es bekannt, eine Aufnahme der jeweiligen Hautläsion beispielsweise mit einem an sich bekannten Videodermatoskop zu erstellen und diese für eine Evaluierung durch den jeweiligen behandelnden Arzt in entsprechenden Ausgabemitteln in Form von Einzelbildern darzustellen und für einen späteren Vergleich oder Auswertung digital abzuspeichern.

Weiterer Stand der Technik ist aus der US 2002 / 010 394 A1, der US 9 427 187 B2, der US 2010 / 302 358 A1 und der US 2018 / 061 046 A1 bekannt.

Der vorliegenden Erfindung liegt die Aufgabe zugrunde, die bekannten Vorrichtungen und Verfahren weiter zu verbessern. Insbesondere soll eine optimierte bildgebende Vorrichtung zur Erfassung und Charakterisierung einer Hautläsion bereitgestellt werden, welche verbesserte Erkennung von bösartigem und gutartigem Hautgewebe durch den behandelnden Arzt ermöglicht. Diese Aufgabe wird durch den Gegenstand der unabhängigen Ansprüche gelöst. Die Unteransprüche stellen vorteilhafte Weiterbildungen der vorliegenden Erfindung dar. Die Erfindung adressiert zudem weitere Probleme, welche in der folgenden Beschreibung näher erörtert werden.

In einem ersten Aspekt betrifft die Erfindung eine Vorrichtung zur Erzeugung einer Mehrzahl von Abbildungen zur Unterstützung bei der Charakterisierung von Hautläsionen, aufweisend eine Erfassungseinheit, insbesondere ein Videodermatoskop, zur selektiven Beleuchtung und Erfassung von Aufnahmen eines eine Hautläsion aufweisenden Hautbereichs, wobei die Erfassungseinheit eine Vergrößerungsoptik aufweist, welche mindestens eine 160-fache, bevorzugt eine mindestens 240-fache optische Vergrößerung des Hautbereichs zur Erfassung einzelner morphologischer Strukturen der Hautläsion bereitstellt, eine der Vergrößerungsoptik zugeordnete Fokussiereinheit, welche ausgebildet ist, eine Fokusebene der Vergrößerungsoptik ausgehend von einer Hautoberfläche des Hautbereichs vorzugsweise schrittweise in eine Mehrzahl an unterschiedlichen Tiefenebenen unterhalb der Hautoberfläche zu legen, eine Steuerungseinheit, welche zur Ansteuerung der Fokussiereinheit und/oder der Erfassungseinheit ausgebildet ist und diese vorzugsweise derart ansteuert, dass bei Anordnung der Fokusebene in einer jeweiligen Tiefenebene eine Aufnahmenerfassung, vorzugsweise als Video- und/oder Einzelbilderfassung, durch die Erfassungseinheit erfolgt, und eine Verarbeitungs- und Ausgabeeinheit zur Abbildungserzeugung basierend auf den von der Erfassungseinheit bereitgestellten Bildinformationen. Die Verarbeitungs- und Ausgabeeinheit ist ausgebildet, eine jeweilige Detailabbildung morphologischer Strukturen der Hautläsion in einer jeweiligen Tiefenebene als Live-Videobild und/oder als Einzelabbildung in Kombination mit einem variierbaren Steuerelement zur Darstellung und/oder Adaption der jeweiligen Tiefenebene und/oder in Kombination mit einem erfassten Referenzbild der Hautläsion darzustellen, wobei die Fokussiereinheit ausgebildet ist, automatisch oder manuell, die Fokusebene selektiv in eine Tiefenebene von 1 bis 50µm, unterhalb der Hautoberfläche zu legen.

Die vorliegende Vorrichtung ermöglicht - im Gegensatz zur klassischen Dermatoskopie - mit Hilfe der stark vergrößernden Optik, der zugeordneten Fokussiereinheit und einer damit verbundenen Erfassungseinheit, vorzugsweise in Form einer Video- oder Digitalkamera, insbesondere eines Videodermatoskops, die optische Erfassung und anschließende Abbildungserzeugung bzw. Visualisierung von morphologischen Strukturen, insbesondere von Kapillaren und Zellstrukturen in verschiedenen Tiefenebenen einer zu untersuchenden Hautläsion. Die so erzeugten Abbildungen sind vergleichbar mit Schnittbildern, wie sie in der Histologie mit Hilfe eines Mikroskops angefertigt werden. Im Unterschied hierzu ermöglicht die erfindungsgemäße Vorrichtung jedoch die unmittelbare Gewinnung von Farbbildern der morphologischen Strukturen, ohne die Notwendigkeit der Entnahme von Gewebeproben und Untersuchung mit einem Objektträger unter einem Lichtmikroskop. Zudem ermöglicht die vorliegende Erfindung die Bereitstellung von farbigen Abbildungen der morphologischen Strukturen ohne Färbemethoden, welche üblicherweise bei einer histologischen Untersuchung angewandt werden. Die bereitgestellten Dateilabbildungen als Video- und/oder Einzelbildabbildung ermöglichen einem behandelnden Arzt eine detaillierte Analyse der morphologischen Strukturen von Hautläsionen. Insbesondere werden hierbei morphologische (Zell)Strukturen in verschiedenen Tiefenebenen einer Hautläsion sichtbar gemacht bzw. dargestellt, welche vom behandelnden Arzt unmittelbar ausgewertet werden können und gegenüber der klassischen Dermatoskopie zusätzliche Diagnosemöglichkeiten einer Läsion bereitstellen.

Die Vergrößerungsoptik der Erfassungseinheit ermöglicht vorzugsweise eine mindestens 160 bis 400-fache, mehr bevorzugt eine mindestens 240 bis 400-fache und weiter bevorzugt eine mindestens 300 bis 400-fache optische Vergrößerung des zu untersuchenden Hautbereichs bzw. einer zu untersuchenden Läsion im Hautbereich. Die Vergrößerungsoptik und die zugeordnete Fokussiereinheit können dabei in einem vorzugsweise austauschbaren Vorsatzmodul für ein Kameramodul der Erfassungseinheit integriert sein. Die Erfassungseinheit ist vorzugsweise als mobiles bzw. tragbares Handgerät ausgebildet.

Die Fokussiereinheit kann manuelle und/oder automatische Verstellmittel umfassen, welche eine jeweilige Fokusebene der Vergrößerungsoptik selektiv variieren. Die Fokussiereinheit ist vorzugsweise ausgebildet einen Abstand von Linsen der Vergrößerungsoptik zueinander und/oder einen Abstand einer Linsengruppe zum Kameramodul der Erfassungseinheit zu variieren. Die Verstellmittel können beispielsweise einen Elektromotor und/oder einen Mikro-Aktuator umfassen. Eine manuelle oder automatische Verstellung kann beispielsweise mithilfe eines manuell betätigbaren Steuerungselements, beispielsweise einem Drehring oder einem Dreh- und/oder Schaltknopf, mithilfe der Steuerungseinheit der Vorrichtung und/oder mithilfe der Verarbeitungs- und Ausgabeeinheit erfolgen. Dabei kann ein Anwender die gewünschte Fokusebene unterhalb der Hautoberfläche im µm-Bereich einstellen.

Die Fokussiereinheit ist vorzugsweise derart ausgebildet, eine Oberfläche des zu untersuchenden Hautbereichs bzw. einer Läsion im Rahmen einer Initialisierung, beispielsweise bei oder kurz nach einer Positionierung an der Läsion, insbesondere bei einem Aufsetzen der Erfassungseinheit auf den die Läsion aufweisenden Hautbereich, vorzugsweise automatisch zu Fokussieren.

Die Fokussiereinheit ist erfindungsgemäß ausgebildet, neben einer Fokussierung der Vergrößerungsoptik auf eine Hautoberfläche bzw. eine Läsionsoberfläche die Fokusebene selektiv in eine Tiefenebene von 1 bis 50µm, mehr bevorzugt 5 bis 50µm, unterhalb der Hautoberfläche zu legen. In anderen Worten erfolgt hierbei eine vorzugsweise selektive Fokussierung einer Tiefenebene, welche 1 bis 50µm, mehr bevorzugt 5 bis 50µm unterhalb einer vorzugsweise initial fokussierten Hautoberfläche liegt. Es können somit detaillierte (Zell)strukturaufnahmen bis in eine Tiefe von 50µm unterhalb der Hautoberfläche erfasst und durch die Verarbeitungs- und Ausgabeeinheit abgebildet werden. Hierbei können tiefenabhängige Aufnahmen bzw. Abbildungen der morphologischen Strukturen der Läsion erfasst und zur Analyse durch den behandelnden Arzt abgebildet werden.

Die Fokussiereinheit ist vorzugsweise ausgebildet die Fokusebene in Schritten von 1 bis 7µm, vorzugsweise in Schritten von 2,5 bis 5µm, insbesondere unterhalb der Hautoberfläche, automatisch oder manuell zu variieren.

Die Steuerungseinheit ist vorzugsweise zur Ansteuerung der Fokussiereinheit und/oder der Erfassungseinheit derart ausgebildet, dass in einer jeweiligen Tiefenebene eine Abbildungs- bzw. Aufnahmenerfassung und/oder Speicherung der jeweiligen Abbildung erfolgt.

Die Steuerungseinheit ist vorzugsweise zur Ansteuerung der Fokussiereinheit und/oder der Erfassungseinheit derart ausgebildet, dass - vorzugsweise nach einer Initialisierung bzw. Fokussierung auf die Hautoberfläche - eine vordefinierte Anzahl an Tiefenebenen und/oder vordefinierte diskrete Tiefenebenen selektiv und/oder in einer Sequenz fokussiert werden und eine entsprechende Erfassung durch die Erfassungseinheit erfolgt. Unter Sequenz wird hierbei eine zeitlich zusammenhängende Ansteuerung verstanden, vorzugsweise innerhalb eines Zeitrahmens von 1 bis 30 Sekunden, mehr bevorzugt innerhalb eines Zeitrahmens von 1 bis 15 Sekunden. Die vordefinierten Tiefenebenen umfassen vorzugsweise wenigstens 2 bis 20, mehr bevorzugt wenigstens 5 bis 15 vordefinierte Tiefenebenen, welche vorzugsweise schrittweise nacheinander zur Abbildungserfassung fokussiert werden. Beispielsweise kann innerhalb einer vordefinierten Sequenz eine Ansteuerung auf die Tiefenebenen bei 5µm, 20µm, 25µm, 30µm unterhalb der Hautoberfläche erfolgen, wobei nach Fokussierung jeder dieser Tiefenebenen eine Abbildungs- bzw. Aufnahmenerfassung erfolgt. Die Steuerungseinheit und/oder die Verarbeitungs- und Ausgabeeinheit können dazu ausgebildet sein, vordefinierte Sequenzen zu erstellen, zu variieren und zu speichern.

Die Erfassungseinheit weist vorzugsweise integrierte oder damit verbindbare Beleuchtungsmittel auf, vorzugsweise eine Vielzahl von insbesondere Weißlicht emittierenden LED Lichtquellen bzw. LED Chips. Die Beleuchtungsmittel sind vorzugsweise zur Beleuchtung der zu untersuchenden der Hautoberfläche mit polarisiertem, insbesondere linear und/oder kreuzpolarisiertem Licht ausgebildet. Die LED Lichtquellen weisen vorzugsweise eine Farbtemperatur von 5400K bis 5600K auf. Der Farbwiedergabeindex Rₐ der LED Lichtquellen liegt vorzugsweise bei wenigstens 92, mehr bevorzugt bei wenigstens 95 und weiter bevorzugt bei wenigstens 97. Die Beleuchtungsmittel sind vorzugsweise zur Beleuchtung der Hautoberfläche bzw. Läsion mittels Auflichtbeleuchtung, bei welcher die Beleuchtungsquelle in einer vordefinierten Distanz zur Hautoberfläche angeordnet ist, und/oder zur Direkteinkopplung von Licht in die Haut, bei welcher die Beleuchtung durch eine direkt an der Hautoberfläche angeordnete Beleuchtungsquelle erfolgt, ausgebildet. Zur Verringerung von Lichtbrechung an der Hautoberfläche kann ein Immersionsgel auf die zu untersuchende Hautoberfläche aufgetragen werden, auf welche dann die Erfassungseinheit bzw. ein Vorsatzmodul der Erfassungseinheit platziert wird.

Die Vorrichtung kann eine Positioniereinheit aufweisen, welche zur selektiven Projektion einer Markierung, insbesondere eines Punkt- oder Kreuzlinienlasers, auf den Hautbereich bzw. auf die in diesem liegende Läsion ausgebildet ist. Die Positioniereinheit kann an der Erfassungseinheit direkt angeordnet sein oder separat dazu ausgebildet sein. Nach der Positionierung der Vergrößerungsoptik auf dem zu untersuchenden Hautbereich kann die Positioniereinheit abgeschaltet werden.

Die Vorrichtung weist vorzugsweise einen RGB-Sensor zur Erfassung der jeweiligen Bildinformationen und darauf basierender Abbildungserzeugung auf. Dieser ist vorzugsweise von einem Kameramodul der Erfassungseinheit umfasst. Die vom RGB-Sensor bereitgestellten Bildinformationen werden von der Verarbeitungs- und Ausgabeeinheit zur Abbildungserzeugung, insbesondere von Farbabbildungen der jeweiligen Tiefenebene verwendet.

Die Vorrichtung kann neben einem RGB-Sensor weitere Sensoren umfassen, beispielsweise einen IR-Sensor, einen multispektralen Sensor zur Detektion von elektromagnetischer Strahlung in wenigstens 4-12 vordefinierten Wellenlängenbereichen oder einen hyperspektralen Sensor zur Detektion von elektromagnetischer Strahlung in wenigstens 8 bis 20 vordefinierten Wellenlängenbereichen. Die Verarbeitungs- und Ausgabeeinheit ist dann vorzugsweise ausgebildet eine oder mehrere Abbildungen basierend auf den RGB-, IR- und/oder multi- oder hyperspektralen Bildinformationen zu erzeugen. Hierbei können zur Erfassung der multi- oder hyperspektralen Bildinformationen zusätzliche Beleuchtungsmittel bereitgestellt sein, insbesondere zusätzliche Infrarot oder UV-(LED)-Lichtquellen.

Die Verarbeitungs- und Ausgabeeinheit der Vorrichtung kann als eine Recheneinheit, insbesondere als Personal Computer (PC) oder Mobilgerät, insbesondere als Laptop, Tablet oder Smartphone, mit zugehöriger Steuerungssoftware ausgebildet sein. Die Steuerungseinheit kann hierbei Teil der Recheneinheit sein. Die Recheneinheit ist mit der Erfassungseinheit kabelgebunden oder kabellos verbunden.

Die Verarbeitungs- und Ausgabeeinheit umfasst vorzugsweise wenigstens eine Speichereinheit und ein Display zur Darstellung der erfassten bzw. erzeugten Abbildungen. Die Verarbeitungs- und Ausgabeeinheit weist für die erfindungsgemäße Darstellung vorzugsweise eine graphische Oberfläche eines Softwareprogramms zur Steuerung der Vorrichtung auf. Die Verarbeitungs- und Ausgabeeinheit umfasst zudem vorzugsweise eine Benutzerschnittstelle, beispielsweise ein Keyboard, eine Maus und/oder ein Touchdisplay zur Steuerung der Verarbeitungs- und Ausgabeeinheit, der Steuerungseinheit oder eines zugeordneten Softwareprogramms. Die Verarbeitungs- und Ausgabeeinheit kann zudem mit wenigstens einem externen Server verbunden sein.

Die Vorrichtung kann zudem eine weitere Erfassungseinheit, insbesondere in Form einer digitalen Fotokamera, und/oder ein weiteres austauschbares Vorsatzmodul für die Erfassungseinheit aufweisen, welches zur Erfassung eines klinischen Übersichtsbilds (Übersichtsbild) des Patienten oder eines Referenz-Übersichtsbilds (Referenzbild) einer zu untersuchenden Hautläsion ausgebildet ist. Dieses weist eine deutlich geringere optische Vergrößerung gegenüber der erfindungsgemäßen Vergrößerungsoptik auf. Als "Übersichtsbild" wird eine Ganz- oder Teilkörperaufnahme eines Patienten verstanden, in welcher wenigstens eine oder eine Mehrzahl zu untersuchender Hautläsionen abgebildet sind. Als "Referenzbild" der zu untersuchenden Hautläsion wird eine Einzelaufnahme einer Hautläsion verstanden, welche die Hautläsion mit einer vorzugsweise 10 bis 25-fachen, mehr bevorzugt einer 15 bis 20-fachen optischen Vergrößerung darstellt.

Erfindungsgemäß kann die Verarbeitungs- und Ausgabeeinheit ausgebildet sein, eine jeweilige Detailabbildung der erfassten morphologischen Strukturen der Hautläsion, insbesondere eine Zellstrukturabbildung, in einer jeweiligen Tiefenebene unter der Hautoberfläche als scharfgestelltes bzw. fokussiertes Live-Videobild und/oder als Einzelabbildung darzustellen. Diese wird vorzugsweise in Kombination mit einem variierbaren Steuerelement zur Darstellung und/oder Adaption der jeweiligen Tiefenebene dargestellt. Ein Anwender kann mittels des Steuerelements somit vorzugsweise unmittelbar die jeweilige Tiefenebene fokussieren bzw. anwählen. Des Weiteren kann ein Anwender bei einem Live-Videobild Momentaufnahmen bzw. sogenannte "Snapshots" erstellen und abspeichern.

Die Verarbeitungs- und Ausgabeeinheit kann hierbei dazu ausgebildet sein insbesondere zum Zwecke der Positionierung der Erfassungseinheit einen Live-Videobild-Modus bereitzustellen. Hierbei wird die fokussierte Hautbereich- bzw. Läsionsoberfläche fokussiert und die erfassten Bildinformationen als Live-Videobild dargestellt bzw. in einem Display der Verarbeitungs- und Ausgabeeinheit ausgegeben. Der Anwender kann hierbei die Hautbereichsoberfläche abtasten bzw. abfahren und die Erfassungseinheit an die gewünschte Stelle zur detaillierteren Analyse positionieren. Wenn die Erfassungseinheit auf die gewünschte Position ausgerichtet ist, kann eine manuelle Umschaltung in einen Einzelbild- bzw. Framemodus erfolgen. Alternativ kann die Verarbeitungs- und Ausgabeeinheit ausgebildet sein, eine stationäre bzw. gleichbleibende Ausrichtung der Erfassungseinheit nach einer initialen Positionierung zu detektieren und automatisch in einen Einzelbild- bzw. Framemodus umzuschalten. In diesem können dann die einzelnen Abbildungen in den jeweiligen Tiefenebenen erfasst werden.

Alternativ kann wie oben beschrieben in der jeweiligen fokussierten Tiefenebene ein Live-Videobild der erfassten Bildinformationen ausgegeben werden. Bei Positionsänderung im Live-Videobildmodus können vom Anwender erstellte Momentaufnahmen innerhalb einer Tiefenebene gespeichert werden, welche sich dann wenigstens teilweise überlagern und welche von der Verarbeitungs- und Ausgabeeinheit mittels eines an sich bekannten Stitching-Algorithmus zu einer zusammenhängenden Einzelabbildung zusammen gefügt werden können.

Die Verarbeitungs- und Ausgabeeinheit kann erfindungsgemäß derart ausgebildet sein, eine die Detailabbildung morphologischer Strukturen der Hautläsion, entweder als Live-Videobild oder als Einzelbilddarstellung, welche in einer jeweiligen Tiefenebene erfasst werden, in Kombination mit einem erfassten Referenzbild der Hautläsion darzustellen. Das Referenzbild stellt die Hautläsion vorzugsweise komplett dar, d.h. die jeweiligen Außenkonturen der einzelnen Hautläsion sind auf dem Bild ersichtlich. Die Kombinationsdarstellung ermöglicht es einem Anwender gleichzeitig die jeweilige Detailabbildung und das zugehörige Referenzbild der Läsion zu analysieren.

Die Verarbeitungs- und Ausgabeeinheit ist vorzugsweise derart ausgebildet, einen Indikator für die jeweilige aktuelle Position der dargestellten Detailabbildung der Hautläsion im Referenzbild darzustellen. Dies kann eine Umrandung wie beispielsweise eine vieleckige, insbesondere rechteckige, Markierung der aktuellen Position der Erfassungseinheit im Referenzbild sein. Bei Änderung der jeweiligen Position der Erfassungseinheit wird dies vorzugsweise durch den Indikator angezeigt bzw. dargestellt. Die jeweilige Erfassung und Darstellung der Position im Referenzbild kann mittels Softwarealgorithmus der Verarbeitungs- und Ausgabeeinheit erfolgen, welcher mittels Bildauswertung eine anfängliche Positionierung der Erfassungseinheit auf der Hautläsion als auch einen entsprechende Bewegung der Erfassungseinheit relativ zur Hautläsion während der Untersuchung verfolgt und für eine Darstellung im Referenzbild berechnet. Für die jeweilige Erfassung können auch zusätzliche Positioniermittel zum Detektieren einer aktuellen Position der Erfassungseinheit auf einer Hautbereich- bzw. Läsionsoberfläche bereitgestellt sein, welche mit der Verarbeitungs- und Ausgabeeinheit verbunden sind.

Die Verarbeitungs- und Ausgabeeinheit ist vorzugsweise ferner ausgebildet, ein (klinisches) Übersichtsbild eines Patienten in Kombination mit der jeweiligen Detailabbildung der Hautläsion und/oder einem erfassten Referenzbild der Hautläsion darzustellen. Das Übersichtsbild weist vorzugsweise einen Indikator für die jeweilige Position der dargestellten Hautläsion im klinischen Übersichtsbild auf. Dies kann eine Umrandung wie beispielsweise eine vieleckige, insbesondere rechteckige, Markierung der aktuellen Position der Läsion im Übersichtsbild sein.

Die Verarbeitungs- und Ausgabeeinheit weist vorzugsweise eine Prozessor- und Speichereinheit (Rechenmittel) umfassend vorzugsweise einen entsprechenden Softwarealgorithmus auf, welche dazu ausgebildet ist, eine Vorcharakterisierung und/oder Bewertung der jeweiligen erfassten Detailabbildung morphologischer Strukturen der Hautläsion, vorzugsweise mittels künstlicher Intelligenz, durchzuführen und eine darauf basierende zugehörige Information, vorzugsweise in Form eines Risikowerts und/oder eine erkannte Hautläsionsklasse, mittels der Verarbeitungs- und Ausgabeeinheit auszugeben. Die Rechenmittel sind hierbei vorzugsweise ausgebildet, die erfassten Detailabbildungen morphologischer Strukturen der jeweiligen Tiefenebenen mittels künstlicher Intelligenz, insbesondere durch ein darauf antrainiertes künstliches neuronales Netzwerk zu analysieren. Dies kann insbesondere darauf antrainiert sein, morphologische Strukturen von nichtmelanozytären und melanozytären Hautläsionen zu unterscheiden. Das künstliche neuronale Netzwerk kann zudem zur Erkennung morphologischer Strukturen einer Mehrzahl von folgenden Typen von Hautläsionen als Klassen ausgebildet sein: Pigmentnävus (melanozytärer Nävus), Dermatofibrom, Malignes Melanom, Aktinische Keratose und Morbus Bowen, Basaliom, seborrhoische Keratose, Altersflecken, Angiom, und/oder Plattenepithelkarzinom

In einer bevorzugten Ausführungsform ist das künstliche neuronale Netzwerk zur Erkennung von vordefinierten Risikoklassen insbesondere hinsichtlich einer Malignität der Hautläsion ausbildet. Hierbei kann eine jeweilige Risikoklasse einen jeweiligen Fortschrittsgrad einer Hautläsion wiederspiegeln. Beispielsweise kann das künstliche neuronale Netzwerk ausgebildet sein, wenigstens zwei, vorzugsweise wenigstens drei unterschiedliche Fortschrittsgrade und damit diesen zugeordnete Risikoklassen einer jeweiligen Hautläsion durch die Analyse der jeweiligen morphologischen Strukturen erkennen. Diese können beispielsweise als leichte, mittlere und hohe Risikoklasse unterschieden werden. Hierbei kann eine höhere Risikoklasse Fortschrittsgrade von Hautläsionen umfassen, welche als für den Menschen riskanter einzustufen bzw. welche eine zeitnahe Behandlung und/oder einen chirurgischen Eingriff benötigen. Weiter bevorzugt ist das künstliche neuronale Netzwerk ausgebildet, zwischen einer Vielzahl von unterschiedlichen Fortschrittsgraden eines Hautläsionstyps zu unterscheiden. Die jeweilige Zuordnung in entsprechende Risikoklassen kann durch das künstliche neuronale Netzwerk selbst und/oder eine der Verarbeitung durch das künstliche neuronale Netzwerk nachgelagerte Berechnung erfolgen.

In einer bevorzugten Ausführungsform erfolgt basierend auf einer jeweiligen erkannten oder berechneten Risikoklasse von Hautläsionstypen und/oder basierend auf einem jeweiligen erkannten Fortschrittsgrad der Hautläsion eine Ausgabe und/oder Berechnung eines vorzugsweise numerischen Risikowerts der jeweiligen Hautläsion. Der numerische Wert liegt vorzugsweise zwischen 0,1 und 1. Hierbei kann ein Wert zwischen 0,1 und 0,2 als niedriger, ein Wert zwischen 0,2 und 0,49 als mittlerer und ein Wert zwischen 0,5 und 1,0 als hoher Risikowert definiert sein.

Die Verarbeitungs- und Ausgabeeinheit ist vorzugsweise ausgebildet, die Vorcharakterisierung und/oder Bewertung der jeweiligen erfassten Detailabbildung darzustellen, insbesondere in Kombination mit dem jeweiligen Referenzbild der Hautläsion. Eine Ausgabe der Vorcharakterisierung und/oder Bewertung, beispielsweise die Darstellung einer Risikoklasse und/oder eines Risikowerts und/oder eine erkannte Hautläsionsklasse erfolgt vorzugsweise in Echtzeit oder ohne wesentliche Zeitverzögerung, d.h. innerhalb 1-20 Sekunden, mehr bevorzugt innerhalb 1-10 Sekunden.

Das künstliche neuronale Netzwerk kann ein an sich bekanntes faltendes neuronales Netzwerk, ein sogenanntes Convolutional Neural Network (CNN), sein. Das künstliche neuronale Netzwerk weist vorzugsweise wenigstens eine verdeckte Schicht, mehr bevorzugt zwischen 1 und 100, am bevorzugtesten zwischen 1 und 20 verdeckte Schichten auf. In einer bevorzugten Ausführungsform weist das künstliche neuronale Netzwerk zwischen 2 und 10.000, vorzugsweise zwischen 2 und 1.000 Neuronen pro Schicht auf.

Das künstliche neuronale Netzwerk ist vorzugsweise ausgebildet, basierend auf mittels überwachten Lernens antrainierten Kenntnissen eine vordefinierte Klassifizierung zu erkennen. Hierbei wird dem künstlichen neuronalen Netzwerk in an sich bekannter Weise durch antrainiertes Lernen eine große Vielzahl von morphologischen Strukturen von Hautläsionen unterschiedlichen Typs, unterschiedlicher Ausbildung und/oder unterschiedlichen Fortschritts entsprechend einem jeweiliger Diagnose vorzugsweise als Bilddaten zum Anlernen bereitgestellt. Diese können insbesondere aus histologischen Untersuchungen bereitgestellt werden. Ein derartiges Anlernen kann in an sich bekannter Weise in einem nachfolgenden Validierungsprozess hinsichtlich der Erkennungsgenauigkeit des antrainierten künstlichen neuronalen Netzwerks überprüft werden. Zudem kann mittels an sich bekannten Transferlernens ein bereits mit einem großen Datenbestand angelerntes künstliches neuronales Netzwerk verwendet werden und unter geringer Parameteränderung auf die jeweilige Verwendungsart angepasst werden. Das Anlernen und Validieren des künstlichen neuronalen Netzwerks kann beispielsweise mit Python Tensorflow und/oder Python Keras erfolgen. Die Bildverarbeitung, Bereitstellung und/oder Zuordnung kann mittels OpenCV2.4 erfolgen.

Das künstliche neuronale Netzwerk kann zudem ausgebildet sein, zuvor angelernte Kenntnisse bei der fortlaufenden Analyse der Hautläsionen aus den zugeführten Bilddaten der morphologischen Strukturen weiter zu verbessern. Das bedeutet, dass das künstliche neuronale Netzwerk vorzugsweise selbstlernend ausgebildet ist und seine Kenntnisse während des fortlaufenden Einsatzes in der Analyse von Abbildungen der morphologischen Strukturen stetig erweitert bzw. verbessert. Hierbei können beispielsweise vom behandelnden Arzt bereitgestellte Informationen hinsichtlich einer jeweiligen Diagnose zu einer erfassten Hautläsion berücksichtigt werden.

In einem weiteren Aspekt betrifft die Erfindung ein Verfahren zur Erzeugung einer Mehrzahl von Abbildungen zur Unterstützung bei der Charakterisierung von Hautläsionen, vorzugsweise mit einer Vorrichtung wie zuvor beschrieben, aufweisend die Schritte:
- Beleuchtung eines zu untersuchenden Hautbereichs durch Beleuchtungsmittel, vorzugsweise durch eine Vielzahl von LED Chips,
- selektive und vorzugsweise schrittweise Fokussierung einer optischen Erfassungseinheit mit zugeordneter Vergrößerungsoptik ausgehend von einer Hautoberfläche auf eine Mehrzahl unterschiedlicher Tiefenebenen unterhalb der Hautoberfläche, wobei die Vergrößerungsoptik mindestens eine 160-fache, bevorzugt eine mindestens 240-fache optische Vergrößerung des Hautbereichs zur Erfassung morphologischer Strukturen einer Hautläsion im Hautbereich ermöglicht,
- Erfassung und vorzugsweise Abspeicherung wenigstens einer Aufnahme der Hautläsion in einer jeweiligen Tiefenebene durch eine Erfassungseinheit,
- Erzeugung wenigstens einer Detailabbildung morphologischer Strukturen der Hautläsion basierend auf den von der Erfassungseinheit bereitgestellten Bildinformationen, und
- Darstellung einer jeweiligen Detailabbildung morphologischer Strukturen der Hautläsion in einer jeweiligen Tiefenebene als Live-Videobild und/oder als Einzelabbildung in Kombination mit einem variierbaren Steuerelement zur Darstellung und/oder Adaption der jeweiligen Tiefenebene und/oder in Kombination mit einem erfassten Referenzbild der Hautläsion durch die Verarbeitungs- und Ausgabeeinheit, wobei bei dem selektiven und vorzugsweise schrittweisen Fokussierung eine Fokusebene automatisch oder manuell in eine Tiefenebene von 1 bis 50µm, unterhalb der Hautoberfläche gelegt wird.

Die Fokussierung in die jeweiligen Tiefenebenen erfolgt vorzugsweise automatisch und schrittweise in vordefinierten Abständen. Diese können vorzugsweise Schritte von jeweils 1 bis 7µm, mehr bevorzugt Schritte von jeweils 2,5 bis 5µm sein. In einer bevorzugten Ausführungsform werden jeweils vordefinierte Tiefenebenen nacheinander in einer Sequenz fokussiert und nach der jeweiligen Fokussierung erfolgt eine Erfassung und Abspeicherung bzw. Sicherung von wenigstens einer Abbildung. Die Erzeugung und/oder Ausgabe einer jeweiligen Detailabbildung durch eine Verarbeitungs- und Ausgabeeinheit erfolgt vorzugsweise als Live-Bild in Echtzeit, insbesondere in einer anfänglichen Initialisierung, im Rahmen welcher die Erfassungseinheit auf die jeweils gewünschte Position der Hautläsion ausgerichtet wird.

Während der Untersuchung der Hautläsion kann durch Positionsänderung der Erfassungseinheit innerhalb einer jeweiligen Tiefenebene ein größerer Bereich analysiert werden. Hierbei entsteht eine Mehrzahl sich mehr oder weniger überlappender Einzelbilder, welche mit einem an sich bekannten Stitching-Algorithmus zu einer zusammenhängenden Abbildung in der jeweiligen Tiefenebene verrechnet werden können. Diese zusammenhängende Abbildung kann dann auf einem Display zur Analyse von einem behandelnden Arzt ausgegeben werden.

Das Verfahren kann zudem die weiteren Schritte einer Erfassung einer zu untersuchenden Hautläsion, vorzugsweise mit einer 10 bis 20-fachen optischen Vergrößerung, und die Darstellung eines Referenzbilds der Hautläsion in Kombination mit einer zugehörigen Detailabbildung der Hautläsion, vorzugsweise nebeneinander auf einem Display einer Verarbeitungs- und Ausgabeeinheit aufweisen. Zudem kann in einem weiteren Schritt ein Indikator dargestellt werden, vorzugsweise im Referenzbild, welcher eine aktuelle Position der dargestellten Detailabbildung in der Hautläsion kennzeichnet.

Das Verfahren kann zudem weitere Merkmale aufweisen, welche im Zusammenhang mit der oben beschriebenen Vorrichtung beschrieben wurden. Zur Vermeidung von Wiederholungen wird auf die obige Beschreibung der Vorrichtung verwiesen. Insbesondere sollen die oben beschriebenen Merkmale auch als für das erfindungsgemäße Verfahren offenbart und beanspruchbar gelten und umgekehrt.

Weitere Vorteile, Merkmale und Einzelheiten der Erfindung ergeben sich aus der nachfolgenden Beschreibung bevorzugter Ausführungsbeispiele sowie anhand der Zeichnungen, diese zeigen in:
- **Fig. 1**: eine schematische Darstellung einer bevorzugten Ausführungsform der erfindungsgemäßen Vorrichtung;
- **Fig. 2**: eine schematische Darstellung der Vergrößerungsoptik und der zugeordneten Fokussiereinheit;
- **Fig. 3**: eine schematische Seitenansicht einer bevorzugten Ausführungsform der Erfassungseinheit als Kameramodul mit einem austauschbaren Vorsatzmodul;
- **Fig. 4**: eine bevorzugte Darstellung bei der erfindungsgemäßen Ausgabe der Abbildung einer Hautläsion mit zugeordneten Informationen;

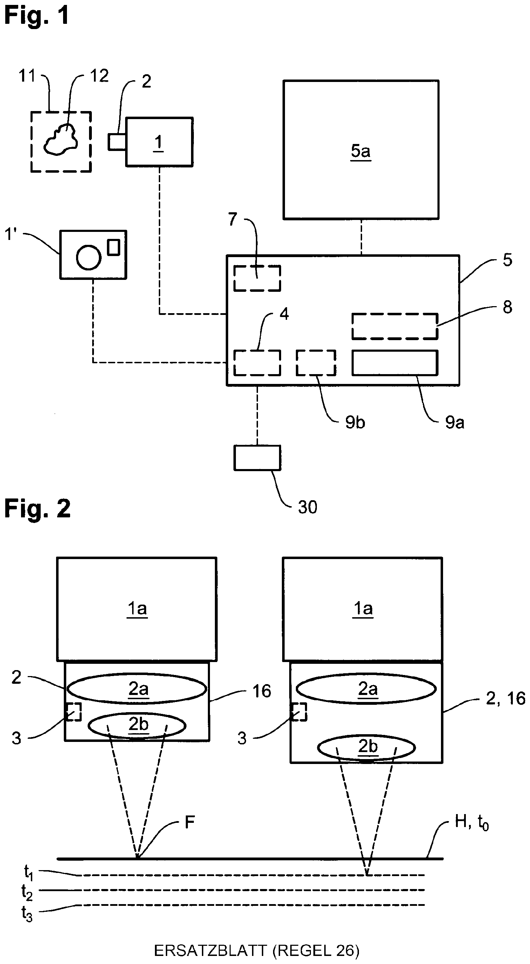

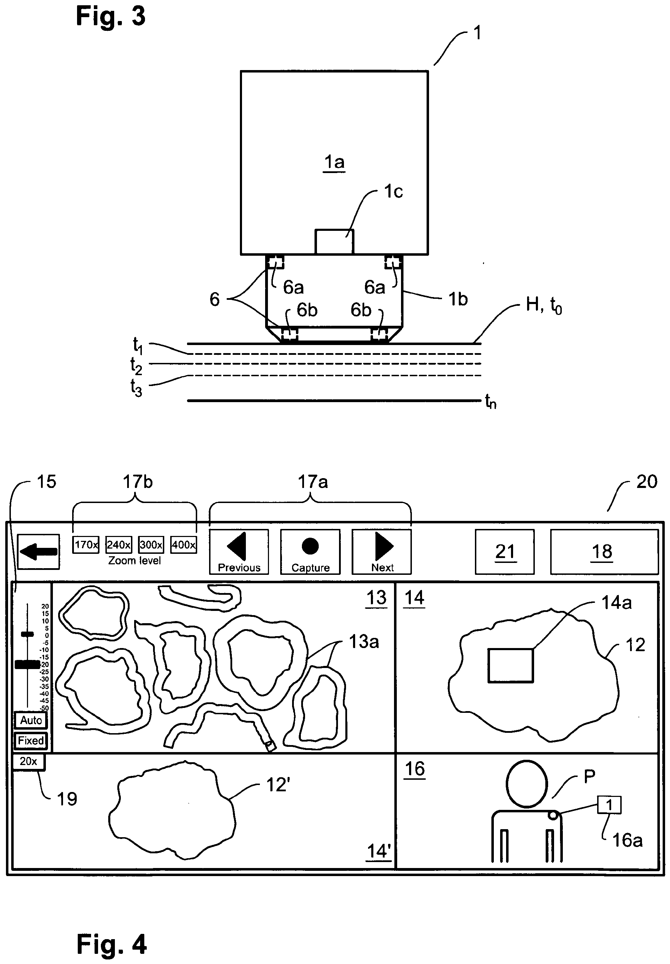

**Fig. 1** zeigt eine bevorzugte Ausführungsform der erfindungsgemäßen Vorrichtung 10. Diese weist eine Erfassungseinheit 1 auf, welche zur Erfassung einer Aufnahme bzw. Abbildung, insbesondere einer optisch stark vergrößerten Detailaufnahme von morphologischen Strukturen 13a einer Hautläsion 12 in einem Hautbereich 11 eines Patienten P (vgl. Fig. 4) ausgebildet ist. Die Erfassungseinheit 1 stellt vorzugsweise digitale Bildinformationen bzw. ein diese repräsentierendes Signal basierend auf der erfassten Aufnahme bereit.

Die Erfassungseinheit 1 umfasst vorzugsweise ein an sich bekanntes Videodermatoskop. Die Erfassungseinheit 1 kann vorzugsweise selektiv in einem Video- oder Einzelbildmodus betrieben werden. Die Erfassungseinheit 1 weist eine Vergrößerungsoption 2 und eine dieser zugeordnete Fokussiereinheit 3 auf und ist vorzugsweise als mobiles Handgerät ausgebildet.

Die Vorrichtung 10 kann optional eine zusätzliche Erfassungseinheit 1' aufweisen. Diese kann eine vorzugsweise hochauflösende digitale Bild- oder Videokamera umfassen und sind zur Erfassung eines Referenzbildes 14 der Hautläsion 12 und/oder eines klinischen Übersichtsbilds 16 des Patienten P ausgebildet.

Die Vorrichtung 10 weist eine Verarbeitungs- und Ausgabeeinheit 5 auf, welche mit der Erfassungseinheit 1,1' verbunden ist. Diese ist zur elektronischen Verarbeitung der bereitgestellten Bildinformationen bzw. Bilddaten und Ausgabe entsprechender Abbildungen ausgebildet. Die Verarbeitungs- und Ausgabeeinheit 5 weist hierfür wenigstens eine Ausgabeeinheit in der Form eines Displays 5a auf. Die Ausgabeeinheit kann zudem zur Bereitstellung von akustischen Signalen oder Warnhinweisen ausgebildet sein.

Eine Steuereinheit 4 der Vorrichtung 10 zur Ansteuerung der Fokussiereinheit 3 und/oder der Erfassungseinheit 1 kann in der Verarbeitungs- und Ausgabeeinheit 5 integriert oder separat ausgebildet sein. Die Verarbeitungs- und Ausgabeeinheit 5 weist zudem eine Prozessor- und Speichereinheit 8 auf. Diese ist vorzugsweise zur Speicherung und Ausführung eines Softwareprogramms zur Steuerung der Vorrichtung ausgebildet. Die Verarbeitungs- und Ausgabeeinheit 5 weist vorzugsweise eine Benutzerschnittstelle 9a zur Steuerung der Vorrichtung 10 und/oder einer darauf ausgeführten Software auf. Diese kann an sich bekannte Eingabemittel wie beispielsweise eine Tastatur, Maus und/oder einen Touchscreen umfassen.

Die Prozessor- und Speichereinheit 8 der Verarbeitungs- und Ausgabeeinheit 5 umfasst in einer bevorzugten Ausführungsform ein vorzugsweise darauf gespeichertes künstliches neuronales Netzwerk, welches zur Erkennung und/oder Klassifizierung von morphologischen Strukturen von Hautläsionen ausgebildet bzw. eingerichtet ist. Hierbei kann das künstliche neuronale Netzwerk auf in der Speichereinheit 8 hinterlegte Daten und/oder auf einen externen Server oder eine externe Speichereinheit 30 zugreifen. Diese kann mittels einer Kommunikationsschnittstelle 9b der Verarbeitungs- und Ausgabeeinheit 5 mit der Prozessor- und Speichereinheit 8 verbunden sein. Die Kommunikationsschnittstelle 9b kann zudem zur Anbindung der Erfassungseinheit 1,1' an die Verarbeitungs- und Ausgabeeinheit 5 ausgebildet sein. Die Kommunikationsschnittstelle 9b kann eine drahtlose und/oder drahtgebundene Kommunikation mit der Erfassungseinheit 1,1` und/oder dem externen Server oder einer externen Speichereinheit 30 ermöglichen.

Die Vorrichtung 10 kann zudem eine Positioniereinheit 7 aufweisen, welche zur selektiven Projektion einer Markierung, insbesondere eines Punkt- oder Kreuzlinienlasers, auf den Hautbereich 11 ausgebildet ist.

**Fig. 2** zeigt eine schematische Darstellung der Vergrößerungsoptik 2 und der zugeordneten Fokussiereinheit 3. Die Vergrößerungsoptik 2 ist hierbei als Vorsatzmodul 1b für ein Kameramodul 1a der Erfassungseinheit 1 ausgebildet und mit diesem vorzugsweise selektiv verbindbar. Die Vergrößerungsoptik 2 ist ausgebildet, eine mindestens 160-fache, mehr bevorzugt eine mindestens 240-fache optische Vergrößerung des Hautbereichs 11 bzw. der Hautläsion 12 bereitzustellen, so dass morphologische Strukturen 13a (vgl. Fig. 4) in verschiedenen Tiefenebenen der Hautläsion 12 erfasst werden können. Die Vergrößerungsoptik umfasst vorzugsweise wenigstens zwei zueinander verstellbare Linsen 2a,2b und/oder eine bezüglich des Kameramoduls 1a verstellbare Linsenbaugruppe.

Die der Vergrößerungsoptik 2 zugeordnete Fokussiereinheit 3 ist vorzugsweise ausgebildet einen Abstand der Linsen 2a,2b der Optik 2 zueinander und/oder einen Abstand einer Linsengruppe zum Kameramodul 1a der Erfassungseinheit 1 zu variieren. Hierzu weist die Fokussiereinheit 3 vorzugsweise Verstellmittel in Form eines Elektromotors und/oder eines Mikro-Aktuators auf. Die Verstellmittel können manuell durch einen Bedienperson und/oder automatisch insbesondere mittels der zugeordneten Steuerungseinheit 4 und/oder die zugeordnete Verarbeitungs- und Ausgabeeinheit 5 gesteuert werden. Wie in Fig. 2 gezeigt, ist die Fokussiereinheit 3 derart ausgebildet, nicht nur die Hautoberfläche H an einer zu untersuchenden Hautläsion 12, welche einer Nulltiefenebene t₀ entspricht, sondern auch selektiv eine Mehrzahl unterschiedlicher Tiefenebenen t₁,...,tₙ unterhalb der Hautoberfläche H, zu fokussieren.

Hierbei ist die Fokussiereinheit 3 vorzugsweise derart ausgebildet, nach einer Positionierung der Erfassungseinheit 1 auf der Hautläsion 12 zunächst im Rahmen einer Initialisierung die Hautoberfläche H vorzugsweise automatisch zu fokussieren. Hierbei wird die Fokusebene F der Vergrößerungsoptik 2 in die entsprechende Nulltiefenebene t₀ gelegt (vgl. linke Darstellung in Fig. 2). Anschließend kann durch einen Anwender manuell und/oder durch die Steuerungseinheit 4 und/oder durch die Verarbeitung- und Ausgabeeinheit 5 eine automatische Einstellung der Fokusebene F auf unterhalb der Hautoberfläche H liegenden Tiefenebenen t₁,...,tₙ erfolgen. In der rechten Darstellung in Fig. 2 ist die Fokusebene F beispielsweise eine erste Tiefenebene t₁ unterhalb der Hautoberfläche H gelegt bzw. diese erste Tiefenebene t₁ fokussiert.

Hierbei können vordefinierte Tiefenebenen t₁,...,tₙ selektiv einzeln oder in einer vordefinierten Sequenz schrittweise nacheinander fokussiert werden, wobei in jeder der Tiefenebenen t₁,...,tₙ eine jeweilige Aufnahmen- bzw. Abbildungserfassung durch die Erfassungseinheit 1 erfolgen kann.

Die Fokussiereinheit 3 ist vorzugsweise ausgebildet, die Fokusebene F selektiv in eine Tiefenebene von 1 bis 50µm, mehr bevorzugt 5 bis 50µm, unterhalb der Hautoberfläche zu legen. Die jeweilige Fokusebene F kann hierbei in Schritten von 1 bis 7µm, mehr bevorzugt in 2,5 bis 5µm automatisch oder manuell variieren werden.

**Fig. 3** zeigt eine schematische Seitenansicht einer bevorzugten Ausführungsform der Erfassungseinheit 1 aufweisend das Kameramodul 1a mit einem austauschbaren Vorsatzmodul 1b. Die Erfassungseinheit 1 weist vorzugsweise integrierte oder damit verbindbare Beleuchtungsmittel 6 auf, vorzugsweise eine Vielzahl von insbesondere Weißlicht emittierenden LED Lichtquellen bzw. LED Chips. Die Beleuchtungsmittel 6 umfassen vorzugsweise endseitig bzw. distal an den Erfassungseinheit 1 angeordnete Beleuchtungsmittel 6b zur Direkteinkopplung von Licht in den zu untersuchenden Hautbereich 11. Diese liegen bei der Untersuchung des Hautbereichs 11 vorzugsweise direkt auf der Hautoberfläche H auf. Zur Verringerung von Lichtbrechung an der Hautoberfläche kann ein Immersionsgel aufgetragen werden, auf welches dann die Erfassungseinheit 1 platziert wird. Die Erfassungseinheit 1 weist optional zusätzlich Auflichtbeleuchtungsmittel 6a auf, welche in einer vordefinierten Distanz zur Hautoberfläche H angeordnet sind. Weiterhin umfasst das Kameramodul 1a eine Sensorik 1c zur Erfassung der Aufnahmen bzw. von Bildinformationen bei der Untersuchung der beleuchteten Läsion 12. Die Sensorik 1c umfasst vorzugsweise wenigstens einen RGB-Sensor. Optional kann die Sensorik weitere Sensoren zur Erfassung von Bildinformationen bei der Untersuchung der Hautläsion 12 in den unterschiedlichen Tiefenebenen t₁,...,tₙ umfassen, insbesondere einen IR-Sensor, einen multi- oder hyperspektralen Sensor.

**Fig. 4** zeigt eine bevorzugte Darstellung bei der erfindungsgemäßen Ausgabe der Abbildung einer Hautläsion 12 mit zugeordneten Informationen mittels eines Displays 5a der Verarbeitungs- und Ausgabeeinheit 5. Diese stellt eine graphische Oberfläche 20 dar, auf welcher wenigstens eine Detailabbildung 13 morphologischer Strukturen 13a, insbesondere Zellstrukturen und/oder Kapillaren in einer vordefinierten Tiefenebene der Hautläsion 12 zur Analyse eines behandelnden Arztes abgebildet werden. Diese kann als Live-Videobild oder als Einzelabbildung dargestellt werden.

Die graphische Oberfläche 20 wird vorzugsweise durch eine Steuerungssoftware der Verarbeitungs- und Ausgabeeinheit 5 bereitgestellt. Die graphische Oberfläche 20 umfasst vorzugsweise Patienteninformationen 18 wie beispielsweise Namen und Alter der zu untersuchenden Person. Die graphische Oberfläche 20 umfasst zudem Steuerungselemente 17, insbesondere Elemente zur Darstellungs- bzw. Aufnahmesteuerung 17a und zur selektiven Steuerung einer Vergrößerung durch die Vergrößerungsoptik 17b auf.

Neben der Detailabbildung 13 weist die graphische Oberfläche 20 vorzugsweise ein Feld 21 zur Darstellung einer Vorcharakterisierung und/oder Bewertung der aktuell erfassten Läsion 12 auf. Hierbei kann insbesondere eine Risikoklasse, ein vorzugsweise numerischer Risikowert und/oder eine erkannte Hautläsionsklasse dargestellt werden. Diese können wie zuvor beschrieben basierend auf der Auswertung der erfassten Aufnahmen mittels künstlicher Intelligenz errechnet bzw. bestimmt werden.

Die graphische Oberfläche 20 umfasst vorzugsweise ein der Detailabbildung 13 zugeordnetes Steuerelement 15, welches zur Anzeige und/oder Adaption der jeweiligen Tiefenebene t₁,...,tₙ dient. Im letzteren Fall kann ein Anwender durch Verschieben des jeweiligen Steuerelements 15 die fokussierte Tiefenebene der Erfassungseinheit 1 selektiv und vorzugsweise in vordefinierten Schritten verändern. Das Steuerelement 15 weist hierbei eine Skala mit vordefinierten Schritten zur Fokusänderung in der Einheit "µm", vorzugsweise von + 20µm bis - 50µm auf. Das Steuerelement 15 kann zudem eine Einstellungsfunktion für eine automatische ("auto") oder manuelle ("fixed") Fokussierung umfassen.

Die graphische Oberfläche 20 umfasst weiterhin ein Referenzbild 14 der Hautläsion 12, welches eine deutlich geringere optische Vergrößerung aufweist und in welchem die Hautläsion 12 komplett dargestellt wird. Das Referenzbild 14 weist vorzugsweise einen Indikator 14a auf, welcher die aktuelle Position der Detailabbildung 13 in der dargestellten Hautläsion 12 darstellt. Eine Positionsänderung der Erfassungseinheit 1 auf der Hautläsion 12 wird vorzugsweise in Echtzeit im Referenzbild 14 dargestellt.

Zusätzlich kann die graphische Oberfläche 20 eine weitere Darstellung eines Referenzbilds 14' umfassen. Dieses kann dem zuvor genannte Referenzbild 14 entsprechend, aber beispielsweise hinsichtlich Farbdarstellung und/oder Größe durch den Anwender veränderbar dargestellt sein. Das weitere Referenzbild 14' kann hierfür zugeordnete Abbildungsinformation 19, beispielsweise einen Vergrößerungswert, umfassen.

Die graphische Oberfläche 20 umfasst vorzugsweise zusätzlich ein klinisches Übersichtsbild 16 des Patienten P. Das Übersichtsbild weist vorzugsweise einen Indikator 16a auf, welcher die jeweilige Position der dargestellten Hautläsion 12 darstellt.

### Bezugszeichenliste

- 1: Erfassungseinheit
- 1a: Kameramodul
- 1b: Vorsatzmodul
- 1c: Kamera-Sensorik
- 2: Vergrößerungsoptik
- 3: Fokussiereinheit
- 4: Steuerungseinheit
- 5: Verarbeitungs- und Ausgabeeinheit
- 5a: Display
- 6: Beleuchtungsmittel
- 6a: Auflichtbeleuchtungsmittel
- 6b: Direkteinkopplungsbeleuchtungsmittel
- 7: Positioniereinheit
- 8: Prozessor- und Speichereinheit (Rechenmittel)
- 9a: Benutzerschnittstelle
- 9b: Kommunikationsschnittstelle
- 10: Vorrichtung
- 11: Hautbereich
- 12: Läsion
- 13: Detailabbildung morphologischer Strukturen
- 13a: morphologische Strukturen (Zellstrukturen, Kapillaren)
- 14: Referenz-Übersichtsbild (Übersichtsabbildung)
- 14a: Indikator Positionsbestimmung Detailabbildung
- 15: Steuerelement Tiefenebene
- 16: klinisches Übersichtsbild
- 16a: Indikator Positionsbestimmung Läsion
- 17: Navigations- und/oder Steuerungselemente
- 17a: Darstellungs- /Aufnahmesteuerung
- 17b: Vergrößerungssteuerung
- 18: Patienteninformation
- 19: Abbildungsinformationen
- 20: graphische Oberfläche
- 21: Ausgabe Risikowert und/oder erkannte Hautläsionsklasse
- 30: externer Server
- t₁,...,tₙ: Tiefenebenen
- t₀: Nulltiefenebene (Hautoberfläche)
- F: Fokusebene
- H: Hautoberfläche
- P: Patient

## Patentansprüche

1. Vorrichtung (10) zur Erzeugung einer Mehrzahl von Abbildungen zur Unterstützung bei der Charakterisierung von Hautläsionen, aufweisend
eine Erfassungseinheit (1), insbesondere ein Videodermatoskop, zur selektiven Beleuchtung und Erfassung von Aufnahmen eines eine Hautläsion (12) aufweisenden Hautbereichs (11), wobei die Erfassungseinheit (1) eine Vergrößerungsoptik (2) aufweist, welche
eine optische Vergrößerung des Hautbereichs (11) zur Erfassung morphologischer Strukturen der Hautläsion (12) bereitstellt,
eine der Vergrößerungsoptik (2) zugeordnete Fokussiereinheit (3), welche ausgebildet ist, eine Fokusebene (F) der Vergrößerungsoptik (2) ausgehend von einer Hautoberfläche (t₀) des Hautbereichs (11) vorzugsweise schrittweise in eine Mehrzahl an unterschiedlichen Tiefenebenen (t₁...tₙ) unterhalb der Hautoberfläche zu legen,
eine Steuerungseinheit (4), welche zur Ansteuerung der Fokussiereinheit (3) und/oder der Erfassungseinheit (1) ausgebildet ist und diese vorzugsweise derart ansteuert, dass bei Anordnung der Fokusebene (F) in einer jeweiligen Tiefenebene (t₁...tₙ) eine Aufnahme durch die Erfassungseinheit (1) erfolgt,
eine Verarbeitungs- und Ausgabeeinheit (5) zur Abbildungserzeugung basierend auf den von der Erfassungseinheit (1) bereitgestellten Bildinformationen; und
wobei die Verarbeitungs- und Ausgabeeinheit (5) ausgebildet ist, eine jeweilige Detailabbildung (13) morphologischer Strukturen der Hautläsion (12) in einer jeweiligen Tiefenebene (t₁...tₙ) als Live-Videobild und/oder als Einzelabbildung in Kombination mit einem variierbaren Steuerelement (15) zur Darstellung und/oder Adaption der jeweiligen Tiefenebene (t₁...tₙ) und/oder in Kombination mit einem erfassten Referenzbild (14) der Hautläsion (12) darzustellen, **gekennzeichnet dadurch, dass**
die Vergrößerungsoptik mindestens eine 160-fache, bevorzugt eine mindestens 240-fache optische Vergrößerung des Hautbereichs (11) zur Erfassung morphologischer Strukturen der Hautläsion (12) bereitstellt und die Fokussiereinheit (3) ausgebildet ist, automatisch oder manuell, die Fokusebene (F) selektiv in eine Tiefenebene (t₁...tₙ) von 1 bis 50µm, unterhalb der Hautoberfläche (t₀) zu legen.

2. Vorrichtung nach Anspruch 1,
wobei die Vergrößerungsoptik (2) eine 250 bis 400-fache optische Vergrößerung des Hautbereichs (11) ermöglicht.

3. Vorrichtung nach einem der Ansprüche 1 bis 2,
wobei die Fokussiereinheit (3) ausgebildet ist, die Fokusebene (F) in 1 bis 7µm Schritten, vorzugsweise in 2,5 bis 5µm Schritten automatisch oder manuell zu variieren.

4. Vorrichtung nach einem der vorhergehenden Ansprüche,
wobei die Steuerungseinheit (4) zur Ansteuerung der Fokussiereinheit (3) und/oder der Erfassungseinheit (1) derart ausgebildet ist, dass eine Vielzahl vordefinierter Tiefenebenen (t₁...tₙ) in einer Sequenz nacheinander fokussiert werden und jeweils mindestens eine Aufnahme erfasst wird.

5. Vorrichtung nach einem der vorhergehenden Ansprüche,
wobei die Vergrößerungsoptik (2) und die zugeordnete Fokussiereinheit (3) in einem austauschbaren Vorsatzmodul (1b) für ein Kameramodul (1a) einer Erfassungseinheit (1), insbesondere eines Videodermatoskops, integriert sind.

6. Vorrichtung nach einem der vorhergehenden Ansprüche,
wobei die Erfassungseinheit (1) integrierte oder damit verbindbare Beleuchtungsmittel (6), vorzugsweise eine Vielzahl von LED Lichtquellen, zur Auflichtbeleuchtung (6a) und/oder Direkteinkopplung von Licht (6b) in den Hautbereich (2) aufweist.

7. Vorrichtung nach einem der vorhergehenden Ansprüche,
wobei die Vorrichtung (10) eine Positioniereinheit (7) aufweist, welche zur selektiven Projektion einer Markierung, insbesondere eines Punkt- oder Kreuzlinienlasers, auf den Hautbereich ausgebildet ist.

8. Vorrichtung nach Anspruch 1 bis 7,
wobei die Verarbeitungs- und Ausgabeeinheit (5) ausgebildet ist, einen Indikator (14a) für die jeweilige aktuelle Position der dargestellten Detailabbildung (13) der Hautläsion (12) im Referenzbild (14) darzustellen.

9. Vorrichtung nach einem Ansprüche 1 bis 8,
wobei die Verarbeitungs- und Ausgabeeinheit (5) ausgebildet ist, ein klinisches Übersichtsbild (16) eines Patienten (P) in Kombination mit der jeweiligen Detailabbildung (13) der Hautläsion (12) und/oder einem erfassten Referenzbild (14) der Hautläsion (12) darzustellen, wobei das klinische Übersichtsbild (16) einen Indikator (16a) für die jeweilige Position der dargestellten Hautläsion (12) im klinischen Übersichtsbild (16) aufweist.

10. Vorrichtung nach einem der vorhergehenden Ansprüche,
wobei die Vorrichtung eine Prozessor- und Speichereinheit (8) aufweist, welche dazu ausgebildet ist, eine Vorcharakterisierung und/oder Bewertung der erfassten Detailabbildung (13) morphologischer Strukturen des Hautbereichs, vorzugsweise mittels künstlicher Intelligenz, durchzuführen und eine darauf basierende zugehörige Information, vorzugsweise in Form eines Risikowerts, mittels der Verarbeitungs- und Ausgabeeinheit (5) auszugeben.

11. Verfahren zur Erzeugung einer Mehrzahl von Abbildungen zur Unterstützung bei der Charakterisierung von Hautläsionen, vorzugsweise mit einer Vorrichtung (10) nach einem der Ansprüche 1-10, aufweisend die Schritte:
Beleuchtung eines zu untersuchenden Hautbereichs (11) durch Beleuchtungsmittel (6), vorzugsweise eine Vielzahl von LED Chips,
selektive und vorzugsweise schrittweise Fokussierung einer optischen Erfassungseinheit (1) mit zugeordneter Vergrößerungsoptik (2) ausgehend von einer Hautoberfläche (H) auf eine Mehrzahl unterschiedlicher Tiefenebenen (t₁...tₙ) unterhalb der Hautoberfläche (H), wobei die Vergrößerungsoptik (2) eine optische Vergrößerung des Hautbereichs (11) zur Erfassung morphologischer Strukturen einer Hautläsion (12) im Hautbereich (11) ermöglicht,
Erfassung wenigstens einer Aufnahme der Hautläsion (12) in einer jeweiligen Tiefenebene (t₁...tₙ) durch eine Erfassungseinheit (1),
Erzeugung wenigstens einer Detailabbildung (13) morphologischer Strukturen der Hautläsion (12) basierend auf den von der Erfassungseinheit (1) bereitgestellten Bildinformationen, und
Darstellung einer jeweiligen Detailabbildung (13) morphologischer Strukturen der Hautläsion (12) in einer jeweiligen Tiefenebene (t₁...tₙ) als Live-Videobild und/oder als Einzelabbildung in Kombination mit einem variierbaren Steuerelement (15) zur Darstellung und/oder Adaption der jeweiligen Tiefenebene (t₁...tₙ) und/oder in Kombination mit einem erfassten Referenzbild (14) der Hautläsion (12) durch die Verarbeitungs- und Ausgabeeinheit (5), **gekennzeichnet dadurch, dass**
die Vergrößerungsoptik mindestens eine 160-fache, bevorzugt eine mindestens 240-fache optische Vergrößerung des Hautbereichs (11) zur Erfassung morphologischer Strukturen der Hautläsion (12) ermöglicht und bei dem selektiven und vorzugsweise schrittweisen Fokussierung eine Fokusebene (F) automatisch oder manuell in eine Tiefenebene (t₁...tₙ) von 1 bis 50µm, unterhalb der Hautoberfläche (t₀) gelegt wird.

12. Verfahren nach Anspruch 11,
wobei die Fokussierung in die jeweiligen Tiefenebenen automatisch und schrittweise in vordefinierten Abständen, von vorzugsweise 1 bis 7µm, mehr bevorzugt von 2,5 bis 5µm, erfolgt.

13. Verfahren nach Anspruch 11 oder 12,
wobei jeweils vordefinierte Tiefenebenen (t₁...tₙ) nacheinander in einer Sequenz fokussiert werden und nach der jeweiligen Fokussierung eine Erfassung und Speicherung von wenigstens einer Aufnahme erfolgt.

14. Verfahren nach einem der Ansprüche 11 bis 13, aufweisend die folgenden weiteren Schritte:
Erfassung einer zu untersuchenden Hautläsion (12), und
Darstellung eines Referenzbilds (14) der Hautläsion (12) in Kombination mit einer zugehörigen Detailabbildung (13) der Hautläsion (12), vorzugsweise nebeneinander auf einem Display (5a) einer Verarbeitungs- und Ausgabeeinheit (5).

15. Verfahren nach Anspruch 14,
aufweisend den weiteren Schritt der Darstellung eines Indikators (14a), vorzugsweise im Referenzbild (14), zur Kennzeichnung einer aktuellen Position der dargestellten Detailabbildung (13) in der Hautläsion (12).

## Claims

1. A device (10) for producing a plurality of images for assisting in characterizing skin lesions, the device comprising
a capturing unit (1), in particular a video dermatoscope, for selectively illuminating and capturing recordings of an area of skin (11) presenting a skin lesion (12), the capturing unit (1) comprising magnifying optics (2) providing optical magnification of the area of skin (11) for capturing morphological structures of the skin lesion (12);
a focusing unit (3) associated with the magnifying optics (2) and configured to place a focal plane (F) of the magnifying optics (2) in a plurality of different depth planes (t₁, ..., tₙ) below a skin surface (t₀) of the area of skin (11) starting from the skin surface, preferably in steps;
a control unit (4) configured to control the focusing unit (3) and/or the capturing unit (1) and preferably controlling them in such a manner that the capturing unit (1) captures a recording when the focal plane (F) is located in a respective depth plane (t₁, ..., tₙ);
a processing and output unit (5) for image production based on the image information provided by the capturing unit (1); and
the processing and output unit (5) being configured to display a respective detail image (13) of morphological structures of the skin lesion (12) in a respective depth plane (t₁, ..., tₙ) as a live video image and/or as an individual image in combination with a variable control element (15) for displaying and/or adapting the respective depth plane (t₁, ..., tₙ) and/or in combination with a captured reference image (14) of the skin lesion (12), **characterized in that** the magnifying optics provides at least 160 x, preferably at least 240 x optical magnification of the area of skin (11) for capturing morphological structures of the skin lesion (12), and the focusing unit (3) is configured to selectively place the focal plane (F), automatically or manually, in a depth plane (t₁, ..., tₙ) of 1 to 50 µm below the skin surface (t₀).

2. The device according to claim 1,
wherein the magnifying optics (2) allows 250 x to 400 x optical magnification of the area of skin (11).

3. The device according to claim 1 or 2,
wherein the focusing unit (3) is configured to automatically or manually vary the focal plane (F) in steps of 1 to 7 µm, preferably in steps of 2.5 to 5 µm.

4. The device according to any one of the preceding claims,
wherein the control unit (4) is configured to control the focusing unit (3) and/or the capturing unit (1) in such a manner that a plurality of predefined depth planes (t₁, ..., tₙ) are focused one after the other in one sequence and at least one recording is captured in each depth plane.

5. The device according to any one of the preceding claims,
wherein the magnifying optics (2) and the associated focusing unit (3) are integrated in an exchangeable front-end module (1b) for a camera module (1a) of a capturing unit (1), in particular of a video dermatoscope.

6. The device according to any one of the preceding claims,
wherein the capturing unit (1) comprises illuminating means (6), preferably a plurality of LED light sources, for reflected-light illumination (6a) and/or direct coupling in of light (6b) into the area of skin (2), the illuminating means (6) being integrated in or connectable to the capturing unit (1).

7. The device according to any one of the preceding claims,
wherein the device (10) comprises a positioning unit (7) configured to selectively project a marking, in particular a dot or cross-line laser, onto the area of skin.

8. The device according to claims 1 to 7,
wherein the processing and output unit (5) is configured to display an indicator (14a) for the respective current position of the displayed detail image (13) of the skin lesion (12) in the reference image (14).

9. The device according to any one of claims 1 to 8,
wherein the processing and output unit (5) is configured to display a clinical overview image (16) of a patient (P) in combination with the respective detail image (13) of the skin lesion (12) and/or a captured reference image (14) of the skin lesion (12), the clinical overview image (16) containing an indicator (16a) for the respective position of the displayed skin lesion (12) in the clinical overview image (16).

10. The device according to any one of the preceding claims,
wherein the device comprises a processor and memory unit (8) configured to pre-characterize and/or assess the captured detail image (13) of morphological structures of the area of skin, preferably by means of artificial intelligence, and to output an associated information based thereon, preferably in the form of a risk value, by means of the processing and output unit (5).

11. A method for producing a plurality of images for assisting in characterizing skin lesions, preferably using a device (10) according to any one of claims 1 to 10, the method comprising the following steps:
illuminating an area of skin (11) to be examined by means of illuminating means (6), preferably a plurality of LED chips;
selectively focusing an optical capturing unit (1) with associated magnifying optics (2) on a plurality of different depth planes (t₁, ..., tₙ) below a skin surface (H) starting from the skin surface (H), preferably in steps, the magnifying optics (2) allowing optical magnification of the area of skin (11) for capturing morphological structures of a skin lesion (12) in the area of skin (11);
capturing at least one recording of the skin lesion (12) in a respective depth plane (t₁, ..., tₙ) by means of a capturing unit (1);
producing at least one detail image (13) of morphological structures of the skin lesion (12) based on the image information provided by the capturing unit (1); and
displaying a respective detail image (13) of morphological structures of the skin lesion (12) in a respective depth plane (t₁, ..., tₙ) as a live video image and/or as an individual image in combination with a variable control element (15) for displaying and/or adapting the respective depth plane (t₁, ..., tₙ) and/or in combination with a captured reference image (14) of the skin lesion (12) by means of the processing and output unit (5),
**characterized in that** the magnifying optics provides at least 160 x, preferably at least 240 x optical magnification of the area of skin (11) for capturing morphological structures of the skin lesion (12), and the step of selectively focusing, preferably in steps, comprises automatically or manually placing the focal plane (F) in a depth plane (t₁, ..., tₙ) of 1 to 50 µm below the skin surface (t₀).

12. The method according to claim 11,
wherein the focusing into the respective depth planes takes place automatically and in steps at predefined intervals of preferably 1 to 7 µm, more preferably of 2.5 to 5 µm.

13. The method according to claim 11 or 12,
wherein predefined depth planes (t₁, ..., tₙ) are focused one after the other in one sequence and at least one recording is captured and stored after the respective focusing.

14. The method according to any one of claims 11 to 13, the method comprising the following further steps:
capturing a skin lesion (12) to be examined; and
displaying a reference image (14) of the skin lesion (12) in combination with an associated detail image (13) of the skin lesion (12), preferably next to each other on a display (5a) of a processing and output unit (5).

15. The method according to claim 14,
the method comprising the further step of displaying an indicator (14a), preferably in the reference image (14), for marking a current position of the displayed detail image (13) in the skin lesion (12).

## Revendications

1. Dispositif (10) pour produire une pluralité d'images pour assister dans la caractérisation de lésions cutanées, le dispositif comprenant
une unité de capture (1), notamment un vidéodermoscope, pour éclairer et capturer sélectivement des enregistrements d'une région de peau (11) présentant une lésion cutanée (12), l'unité de capture (1) comprenant une optique de grossissement (2) permettant le grossissement optique de la région de peau (11) pour capturer des structures morphologiques de la lésion cutanée (12) ;
une unité de focalisation (3) associée à l'optique de grossissement (2) et configurée pour placer un plan focal (F) de l'optique de grossissement (2) dans une pluralité de plans de profondeur (t₁, ..., tₙ) différents au-dessous d'une surface de peau (t₀) de la région de peau (11) à partir de la surface de peau, de préférence par étapes ;
une unité de commande (4) configurée pour commander l'unité de focalisation (3) et/ou l'unité de capture (1) et, de préférence, pour les commander de telle manière que l'unité de capture (1) capture un enregistrement quand le plan focal (F) est situé dans un plan de profondeur (t₁, ..., tₙ) respectif ;
une unité de traitement et de sortie (5) pour la production d'images sur la base des informations d'image fournies par l'unité de capture (1) ; et
l'unité de traitement et de sortie (5) étant configurée pour afficher une image détaillée (13) respective de structures morphologiques de la lésion cutanée (12) dans un plan de profondeur (t₁, ..., tₙ) respectif comme vidéo en direct et/ou comme image individuelle en combinaison avec un élément de commande (15) variable pour afficher et/ou adapter le plan de profondeur (t₁, ..., tₙ) respectif et/ou en combinaison avec une image de référence (14) capturée de la lésion cutanée (12), **caractérisé en ce que** l'optique de grossissement fournit un grossissement optique de la région de peau (11) d'au moins 160 fois, de préférence d'au moins 240 fois, pour capturer des structures morphologiques de la lésion cutanée (12), et l'unité de focalisation (3) est configurée pour placer sélectivement le plan focal (F), de manière automatique ou manuelle, dans un plan de profondeur (t₁, ..., tₙ) de 1 à 50 µm au-dessous de la surface de peau (t₀) de manière automatique ou manuelle.

2. Dispositif selon la revendication 1,
dans lequel l'optique de grossissement (2) permet un grossissement optique de la région de peau (11) de 250 fois à 400 fois.

3. Dispositif selon la revendication 1 ou 2,
dans lequel l'unité de focalisation (3) est configurée pour varier automatiquement ou manuellement le plan focal (F) par étapes de 1 à 7 µm, de préférence par étapes de 2,5 à 5 µm.

4. Dispositif selon l'une quelconque des revendications précédentes,
dans lequel l'unité de commande (4) est configurée pour commander l'unité de focalisation (3) et/ou l'unité de capture (1) de telle manière qu'une pluralité de plans de profondeur (t₁, ..., tₙ) prédéfinis sont focalisés l'un après l'autre dans une séquence et au moins un enregistrement est capturé dans chaque plan de profondeur.

5. Dispositif selon l'une quelconque des revendications précédentes,
dans lequel l'optique de grossissement (2) et l'unité de focalisation (3) associée sont intégrées dans un module de montage (1b) changeable pour un module de caméra (1a) d'une unité de capture (1), notamment d'un vidéodermoscope.

6. Dispositif selon l'une quelconque des revendications précédentes,
dans lequel l'unité de capture (1) comprend des moyens d'éclairage (6), de préférence une pluralité de sources de lumière DEL, pour l'éclairage à épiluminescence (6a) et/ou le couplage direct de la lumière (6b) dans la région de peau (2), les moyens d'éclairage (6) étant intégrés dans ou configurés pour être liés à l'unité de capture (1).

7. Dispositif selon l'une quelconque des revendications précédentes,
dans lequel le dispositif (10) comprend une unité de positionnement (7) configurée pour projeter sélectivement un marquage, notamment un point ou un laser à lignes croisées, sur la région de peau.

8. Dispositif selon la revendication 1 à 7,
dans lequel l'unité de traitement et de sortie (5) est configurée pour afficher un indicateur (14a) pour la position actuelle respective de l'image détaillée (13) affichée de la lésion cutanée (12) dans l'image de référence (14).

9. Dispositif selon l'une quelconque des revendications 1 à 8,
dans lequel l'unité de traitement et de sortie (5) est configurée pour afficher une image synoptique clinique (16) d'un patient (P) en combinaison avec l'image détaillée (13) respective de la lésion cutanée (12) et/ou une image de référence (14) capturée de la lésion cutanée (12), l'image synoptique clinique (16) contenant un indicateur (16a) pour la position respective de la lésion cutanée (12) affichée dans l'image synoptique clinique (16).

10. Dispositif selon l'une quelconque des revendications précédentes,
dans lequel le dispositif comprend une unité de processeur et de mémoire (8) configurée pour pré-caractériser et/ou évaluer l'image détaillée (13) capturée de structures morphologiques de la région de peau, de préférence au moyen d'une intelligence artificielle, et pour sortir une information associée sur cette base, de préférence en forme d'une valeur de risque, au moyen de l'unité de traitement et de sortie (5).

11. Procédé pour produire une pluralité d'images pour assister dans la caractérisation de lésions cutanées, de préférence en utilisant un dispositif (10) selon l'une quelconque des revendications 1 à 10, le procédé comprenant les étapes consistant à :
éclairer une région de peau (11) à examiner au moyen de moyens d'éclairage (6), de préférence d'une pluralité de chips DEL ;
focaliser sélectivement une unité de capture (1) optique avec une optique de grossissement (2) associée sur une pluralité de plans de profondeur (t₁, ..., tₙ) différents au-dessous d'une surface de peau (H) à partir de la surface de peau (H), de préférence par étapes, l'optique de grossissement (2) permettant le grossissement optique de la région de peau (11) pour capturer des structures morphologiques d'une lésion cutanée (12) dans la région de peau (11) ;
capturer au moins un enregistrement de la lésion cutanée (12) dans un plan de profondeur (t₁, ..., tₙ) respectif au moyen d'une unité de capture (1) ;
produire au moins une image détaillée (13) de structures morphologiques de la lésion cutanée (12) sur la base des informations d'image fournies par l'unité de capture (1) ; et
afficher une image détaillée (13) respective de structures morphologiques de la lésion cutanée (12) dans un plan de profondeur (t₁, ..., tₙ) respectif comme vidéo en direct et/ou comme image individuelle en combinaison avec un élément de commande (15) variable pour afficher et/ou adapter le plan de profondeur (t₁, ..., tₙ) respectif et/ou en combinaison avec une image de référence (14) capturée de la lésion cutanée (12) au moyen de l'unité de traitement et de sortie (5),
**caractérisé en ce que** l'optique de grossissement fournit un grossissement optique de la région de peau (11) d'au moins 160 fois, de préférence d'au moins 240 fois, pour capturer des structures morphologiques de la lésion cutanée (12), et l'étape consistant à focaliser sélectivement, de préférence par étapes, comprend l'étape consistant à placer automatiquement ou manuellement le plan focal (F) dans un plan de profondeur (t₁, ..., tₙ) de 1 à 50 µm au-dessous de la surface de peau (t₀).

12. Procédé selon la revendication 11,
dans lequel la focalisation dans les plans de profondeur respectifs s'effectue automatiquement et par étapes à intervalles prédéfinis, de préférence 1 à 7 µm ou mieux 2,5 à 5 µm.

13. Procédé selon la revendication 11 ou 12,
dans lequel des plans de profondeur (t₁, ..., tₙ) prédéfinis sont focalisés l'un après l'autre dans une séquence et au moins un enregistrement est capturé et enregistré après la focalisation respective.

14. Procédé selon l'une quelconque des revendications 11 à 13, le procédé comprenant en outre les étapes consistant à :
capturer une lésion cutanée (12) à examiner ; et
afficher une image de référence (14) de la lésion cutanée (12) en combinaison avec une image détaillée (13) associée de la lésion cutanée (12), de préférence à côté l'un de l'autre sur un écran (5a) d'une unité de traitement et de sortie (5).

15. Procédé selon la revendication 14,
le procédé comprenant en outre l'étape consistant à afficher un indicateur (14a), de préférence dans l'image de référence (14), pour marquer une position actuelle de l'image détaillée (13) affichée dans la lésion cutanée (12).
